# EUROPEAN PATENT APPLICATION

(11) **EP 2 626 702 A1**
(43) Date of publication of application: **14.08.2013**
(21) Application number: 11830640.6
(22) Date of filing: 04.10.2011
(51) Int. Cl.: G01N 33/53

(54) **DIAGNOSTIC AGENT, DIAGNOSTIC METHOD AND THERAPEUTIC AGENT FOR FIBROMYALGIA**

(30) Priority: 08.10.2010 US 391094 P
(71) Applicant: Axis, Inc., Kagoshima-shi Kagoshima 891-1305 (JP)
(72) Inventor: YAMANO, Yoshihisa, Kagoshima-shi Kagoshima 890-0055 (JP); NISHIOKA, Kusuki, Tokyo 150-0012 (JP)
(74) Representative: Moser & Götze
(86) International application number: PCT/JP2011/072818
(87) International publication number: WO 2012/046708

(57) **Abstract**

The purpose of the present invention is to provide a diagnostic agent and a diagnostic method for appropriately diagnosing fibromyalgia of a specific type, and a therapeutic agent for the aforesaid type. The invention relates to: a diagnostic agent for fibromyalgia relating to an anti-voltage-gated potassium channel complex antibody (anti-VGKC complex antibody), said diagnostic agent comprising a reagent for detecting the anti-VGKC complex antibody; a diagnostic method using the diagnostic agent; and a therapeutic agent for fibromyalgia relating to the anti-VGKC complex antibody. The invention relates to: a diagnostic agent for fibromyalgia relating to an antibody against a voltage-gated potassium channel complex, said diagnostic agent comprising a reagent for detecting the antibody against the voltage-gated potassium channel complex; and a therapeutic agent for fibromyalgia relating to an antibody against voltage-gated potassium channel, said therapeutic agent comprising an effective amount of an anticonvulsant drug (gabapentine or clonazepam) as the active ingredient.

## Description

### Technical Field

The present invention relates to a diagnostic agent, a diagnostic method, and a therapeutic agent for fibromyalgia. More specifically, the present invention is based on new findings that an anti-VGKC (voltage-gated potassium channel) complex antibody is one of the causes of fibromyalgia. Further, the present invention relates to a diagnostic agent and a diagnostic method for diagnosing fibromyalgia relating to the anti-VGKC complex antibody, and to a therapeutic agent for fibromyalgia relating to the anti-VGKC complex antibody.

### Background Art

As a diagnostic method for fibromyalgia, criteria for classification proposed in 1990 by American College of Rheumatology have known. Further, in the following Non-Patent Literature 1, a determination method for fibromyalgia is proposed based on J-FIQ (Japanese version FIQ; Fibromyalgia Impact Questionaire). Furthermore, in Japanese Patent No. 3630689 (Patent Literature 1), there is a disclosure of a method for diagnosing whether a subject has fibromyalgia or not by detecting an anti-polymer antibody.

Fibromyalgia is a disease led by various causes. Therefore, there are many cases in which a therapeutic drug that is effective for a certain patient with fibromyalgia has no efficacy at all in other patients with fibromyalgia. Therefore, a method for not only diagnosing whether a subject has fibromyalgia or not but also appropriately diagnosing the cause and type of fibromyalgia is desired.

On the other hand, as shown in Japanese Patent Application Laid-Open No. 2009-007278 (Patent Literature 2), for a patient affected with a certain type of fibromyalgia, an antidepressant is effective. Further, as is disclosed in this publication, in the treatment for some of the patients with fibromyalgia, an anticonvulsant drug or Chinese herbal medicine is also effective.

In addition, Isaacs syndrome is, for example, a symptom in which convulsion lasts as a result of becoming difficult to control the nervous excitement by the presence of the antibody affecting a potassium channel in vivo. As to Isaacs syndrome, it is known that a selective inhibitor of a voltage-gated potassium channel (VGKC) is effective (for example, Japanese Patent Application National Publication No. 2009-502206).

### Citation List

### Patent Literature

Patent Literature 1: Japanese Patent No. 3630689
Patent Literature 2: Japanese Patent Application Laid-Open No. 2009-007278

### Non-Patent Literature

Non-Patent Literature 1: Health and labor research division "2009 Practice Guideline on Fibromyalgia" Japan Rheumatism Foundation, issued on March 31, 2010, page 58

### Summary of Invention

### Technical Problem

As described above, a method for appropriately diagnosing the cause and type of fibromyalgia, and an establishment of a treatment method depending on the type are desired.

Therefore, one object of the present invention is to provide a diagnostic agent and a diagnostic method, for appropriately diagnosing a certain type of fibromyalgia, and a therapeutic agent for the type.

### Solution to Problem

The present invention is basically based on the findings obtained from Examples, that is, a certain type of fibromyalgia is caused by an antibody against a complex of voltage-gated potassium channel (VGKC) (anti-VGKC complex antibody). Further, the present inventors elucidated that an anticonvulsant drug is effective for the fibromyalgia caused by an anti-VGKC complex antibody.

That is, the first aspect of the present invention relates to a diagnostic agent for fibromyalgia containing a reagent for detecting an anti-VGKC complex antibody. This diagnostic agent is a diagnostic agent for fibromyalgia relating to an antibody against a complex of voltage-gated potassium channel (anti-VGKC complex antibody). Further, the diagnostic agent contains a reagent for detecting an anti-VGKC complex antibody, and thus can elucidate the cause of fibromyalgia for a patient with the fibromyalgia caused by an anti-VGKC complex antibody. Therefore, as described below, appropriate treatment can be applied for this type of fibromyalgia.

The second aspect of the present invention relates to a diagnostic method for fibromyalgia relating to anti-VGKC complex antibody. This method is basically a method for determining whether the cause of the patient with fibromyalgia is the anti-VGKC complex antibody or not by using a diagnostic agent that is described previously. This method, for example, using a reagent for detecting an anti-VGKC complex antibody, determines whether the anti-VGKC complex antibody is contained in the predetermined level or more in a biological sample (for example, serum) of a patient or not.

The third aspect of the present invention relates to a therapeutic agent for fibromyalgia relating to anti-VGKC complex antibody. This therapeutic agent contains an anticonvulsant drug as an active ingredient in an effective amount. Specific anticonvulsant drug is gabapentine or clonazepam. As verified in the following Examples, when an anticonvulsant drug was administered to a patient with fibromyalgia relating to anti-VGKC complex antibody, the symptom of the fibromyalgia was significantly improved. Therefore, a therapeutic agent containing an anticonvulsant drug as an active ingredient is considered to be effective as a therapeutic agent for fibromyalgia relating to anti-VGKC complex antibody. This therapeutic agent is particularly effective as a therapeutic agent used for improvement of the pain for a patient with fibromyalgia having an anti-VGKC complex antibody level of 100 pM or more in a measurement of anti-VGKC complex antibody level using serum.

### Advantageous Effects of Invention

The present invention can provide a diagnostic agent and a diagnostic method, for diagnosing fibromyalgia relating to anti-VGKC complex antibody, and a therapeutic agent for fibromyalgia relating to anti-VGKC complex antibody.

### Brief Description of the Drawings

FIG. 1 is a chart showing an anti-VGKC complex antibody level of each patient with fibromyalgia.

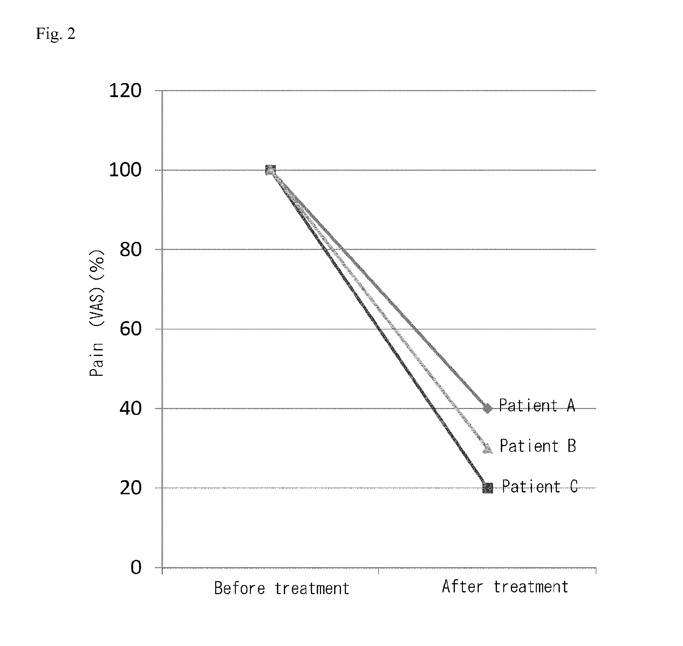

FIG. 2 is a chart showing results of pain-relieving experiments (evaluation by VAS)by an anticonvulsant agent for each anti-VGKC complex antibody positive patient with fibromyalgia.

### Mode for Carrying Out the Invention

A diagnostic agent for fibromyalgia relating to anti-VGKC complex antibody
As described previously, the first aspect of the present invention relates to a diagnostic agent for fibromyalgia containing a reagent for detecting an anti-VGKC complex antibody. This diagnostic agent is a diagnostic agent for fibromyalgia relating to an antibody against a complex of voltage-gated potassium channel. Examples of the anti-VGKC complex antibody include an antibody against a complex of a VGKC and a protein bound to a VGKC, besides the anti-VGKC antibody. The anti-VGKC complex antibody may be an antibody against a complex of a VGKC and a potassium channel inhibitor containing bungarotoxin, or may be an antibody against a complex of a VGKC, a protein bound to a VGKC, and a potassium channel inhibitor containing bungarotoxin. This diagnostic agent can elucidate the cause of fibromyalgia and can provide a guideline for the treatment, for a patient with fibromyalgia in which the cause of fibromyalgia relates to an anti-VGKC complex antibody.

Conventionally, it is known that an anti-VGKC complex antibody relates to Isaacs syndrome of which the number of patients is extremely few. Therefore, the development of a diagnostic agent of anti-VGKC complex antibody has not progressed. On the other hand, as indicated in Examples, the anti-VGKC complex antibody is specifically expressed in a certain patient with fibromyalgia. Therefore, the anti-VGKC complex antibody becomes a specific marker of fibromyalgia. On the other hand, a reagent for directly detecting the anti-VGKC complex antibody has not been developed at this moment. Thus, bungarotoxin that is a potassium channel inhibitor is made to be bound to a VGKC. As a result, a complex consisting of a VGKC and bungarotoxin; or a complex containing a VGKC, a protein bound to a VGKC, and bungarotoxin is obtained. Therefore, by using an antibody against bungarotoxin, a VGKC complex may be detected. That is, the level of VGKC complex may be measured by using the level of the antibody against bungarotoxin. Therefore, by measuring the level of the anti-VGKC complex antibody, the level (that is, the level of VGKC complex in serum) of a protein that forms a VGKC complex by binding to an anti-VGKC antibody or a VGKC is presumed. That is, an example of a reagent for detecting the anti-VGKC complex antibody is a reagent containing bungarotoxin to be bound to a VGKC, an antibody against bungarotoxin, and a reagent for measuring the level of the antibody against bungarotoxin. A reagent containing these is one of the reagents for detecting the anti-VGKC complex antibody of the present invention. Further, a diagnostic agent containing such a reagent for detecting the anti-VGKC complex antibody is effective as a diagnostic agent for fibromyalgia relating to anti-VGKC complex antibody.

A diagnostic method for fibromyalgia relating to anti-VGKC complex antibody
The second aspect of the present invention relates to a diagnostic method for fibromyalgia relating to anti-VGKC complex antibody. This method is basically a method for determining whether the cause of the patient with fibromyalgia is the anti-VGKC complex antibody or not by using a diagnostic agent that is described previously.

This method, for example, using a reagent for detecting an anti-VGKC complex antibody, determines whether the anti-VGKC complex antibody is contained in the predetermined level or more in a biological sample (for example, serum) of a patient or not. In addition, this method may be, described previously, a method in which it is determined whether the anti-VGKC complex antibody is contained in the predetermined level or more or not by forming a complex of VGKC and bungarotoxin, or a complex of a VGKC complex containing VGKC and a certain protein and bungarotoxin, and then by detecting the level of the anti-VGKC complex antibody. Prior to this method, for example, based on J-FIQ, it may be determined whether a subject has fibromyalgia or not. Further, a biological sample is collected from a patient with fibromyalgia, and the level of anti-VGKC complex antibody may be analyzed. In addition, comparing with the results of control experiments, it may be determined whether the level of anti-VGKC complex antibody is the fixed ratio or more or not. An example of the level of anti-VGKC complex antibody that becomes a criterion determining whether the level indicates the fibromyalgia relating to anti-VGKC complex antibody or not is 100 pM.

The biological sample (detection sample) for detecting the level of anti-VGKC complex antibody is not limited to serum. An example of this biological sample except for serum is cerebrospinal fluid. Further, for example, an anti-VGKC complex antibody can be detected by employing an immunological method using an anti-VGKC complex antibody or a VGKC. Examples of the immunological method include Western blotting, dot blotting, slot blotting, ELISA, and RIA. These methods have already known. A VGKC has already known. For example, by using affinity chromatography, or SDS-PAGE, a VGKC is isolated and purified, and a VGKC for detecting the anti-VGKC complex antibody can be obtained.

The anti-VGKC complex antibody is known. Therefore, the present invention can use the known anti-VGKC complex antibody. Further, the anti-VGKC complex antibody may be produced by a method that has already known in the field of biotechnology. For example, an antibody-producing cell that produces anti-VGKC complex antibody is fused with a myeloma cell to establish a hybridoma, and a monoclonal antibody obtained from the hybridoma may be used as the anti-VGKC complex antibody.

A therapeutic agent for fibromyalgia relating to anti-VGKC complex antibody
The third aspect of the present invention relates to a therapeutic agent for fibromyalgia relating to anti-VGKC complex antibody. This therapeutic agent may be, for example, prescribed for a patient who has determined to have fibromyalgia relating to the anti-VGKC complex antibody by a diagnostic method described previously. As the cause of fibromyalgia, various causes can be considered. As verified in the following Examples, a therapeutic agent of the present invention is effective for the treatment of non-depression type of fibromyalgia. An improvement example of such fibromyalgia is an improvement of the pain. The therapeutic agent of the present invention is administered to an anti-VGKC complex antibody positive patient with fibromyalgia (a patient with fibromyalgia having a level of the anti-VGKC complex antibody of 100 pM or more using serum), and is effective for the improvement of the symptom of an anti-VGKC complex antibody positive patient with fibromyalgia.

This therapeutic agent contains an anticonvulsant drug as an active ingredient in an effective amount. Examples of the anticonvulsant drug include gabapentine, phenytoin, mephenytoin, ethotoin, phenobarbital, mephobarbital, primidone, carbamazepine, ethosuximide, methsuximide, phensuximide, valproic acid, trimethadione, paramethadione, phenacemide, acetazolamide, progabide, diazepam, lorazepam, clonazepam, clorazepate salt, and nitrazepam. In the present invention, as verified in the following Examples, gabapentine or clonazepam can be preferably used as an anticonvulsant drug. As verified in the following Examples, when an anticonvulsant drug was administered to a patient with fibromyalgia relating to anti-VGKC complex antibody, the symptom of the fibromyalgia was significantly improved. Therefore, a therapeutic agent containing an anticonvulsant drug as an active ingredient is considered to be effective as a therapeutic agent for fibromyalgia relating to anti-VGKC complex antibody. Hereinafter, the therapeutic agent of the present invention is explained centering on gabapentine and clonazepam.

Gabapentine or clonazepam may be a prodrug. Gabapentine or clonazepam may be a pharmaceutically acceptable salt or solvate.

Chemical name of gabapentine is (1-aminomethyl cyclohexyl)acetic acid. Examples of a pharmacologically acceptable salt of gabapentine include an inorganic acid salt (hydrochloride, sulfate, and the like), an inorganic base salt (sodium salt, potassium salt, calcium salt, and the like), and an organic acid salt (acetate, benzoate, maleate, tartrate, fumarate, mesylate, and the like). Gabapentine or a pharmacologically acceptable salt thereof may be produced according to a conventional method, or may be obtained from the market. An example of commercially available gabapentine is GABAPEN tablet (trade name, active ingredient: gabapentine, Pfizer Inc.).

Examples of clonazepam include "Landsen" manufactured by Dainippon Sumitomo Pharma Co., Ltd., and "Rivotril" manufactured by Chugai Pharmaceutical Co., Ltd. Clonazepam is one kind of benzodiazepine derivatives.

When a drug of the present invention contains gabapentine or clonazepam as an active ingredient, the dosage of the active ingredient may be appropriately determined depending on the weight, the age, the gender, and the degree of the disease of a patient. For examples, the dosage for adult is in the range of 0.5 to 30 mg/day, and preferably 5 to 20 mg/day with oral administration. The dosage per day is preferably administered by dividing into 2 to 4 times. Further, the drug is preferably repeatedly administered, for examples, may be administered continuously for 4 weeks or more.

In the drug of the present invention, the active ingredient is appropriately mixed or diluted/dissolved with preparation carriers such as necessary excipients, disintegrants, binders, lubricants, diluents, buffering agents, tonicity agents, preservatives, wetting agents, emulsifiers, dispersing agents, stabilizing agents, and solubilizing agents, and thus various dosage forms can be produced according to a conventional method. Further, commercially available preparation can be used.

The dosage form of the drug of the present invention is, for example, orally administered preparation such as powders, granules, fine granules, dry syrup, tablet, and capsule, and parenterally administered preparation such as injection, patch, and suppository.

According to the diagnostic method previously described, the present invention also provide a treatment method for fibromyalgia relating to anti-VGKC complex antibody containing: a step of determining whether a subject has fibromyalgia relating to anti-VGKC complex antibody or not; and a step of administering an anticonvulsant drug to the patient who has determined to have fibromyalgia relating to anti-VGKC complex antibody. The patient of this example is a patient with fibromyalgia having an anti-VGKC complex antibody level of 100 pM or more when a VGKC complex was formed by using the serum of the patient. That is, the present invention also provide a treatment method for fibromyalgia containing a step of administering gabapentine or clonazepam to a patient with fibromyalgia having an anti-VGKC complex antibody level of 100 pM or more in a measurement of anti-VGKC complex antibody level using the serum of the patient. Peculiarly the present invention is effective for the improvement of the pain of the patient.

### Example 1

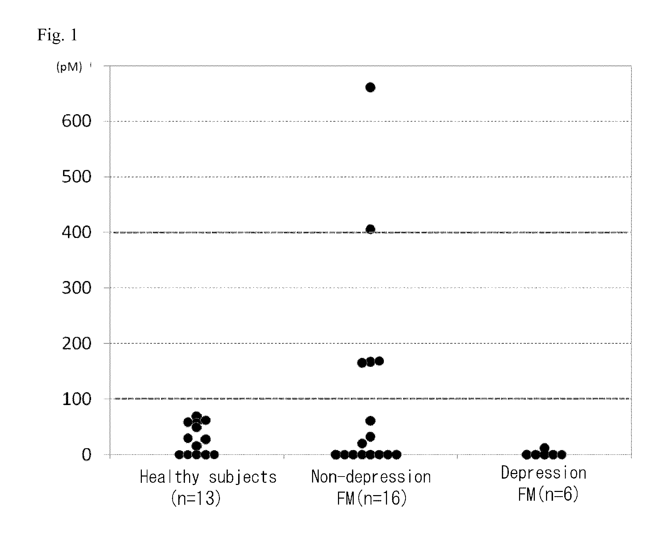

An anti-VGKC complex antibody level in serum of a patient with fibromyalgia
Each serum was collected from 22 patients with fibromyalgia (depression type: 6 patients, and non-depression type: 16 patients) and 13 healthy subjects, a complex with a VGKC or a complex with a VGKC, a certain protein, and bungarotoxin was formed by using bungarotoxin, and then each level of anti-VGKC complex antibody was measured. The level of anti-VGKC complex antibody of 100 pM or more termed positive, 400 pM or more termed most positive. The results were shown in FIG. 1. In examples shown FIG. 1, patients with fibromyalgia termed FM.

In FIG. 1, a complex antibody containing an anti-VGKC complex antibody was not detected from each serum collected from the healthy subjects and the patients with fibromyalgia of depression type. On the other hand, the anti-VGKC complex antibody was detected from each serum collected from 5 patients with fibromyalgia of non-depression type.

Fibromyalgia is a disease of unknown cause. According to these experimental results, as one of the causes of fibromyalgia, an anti-VGKC complex antibody is considered. Therefore, it is considered that by measuring the level of the anti-VGKC complex antibody contained in a patient with fibromyalgia, a cause of an anti-VGKC complex antibody positive patient with fibromyalgia can be elucidated.

### Example 2

Pain-relieving experiments by an anticonvulsant agent for each anti-VGKC complex antibody positive patient with fibromyalgia
It is considered that the pain can be relieved by an anticonvulsant agent for an anti-VGKC complex antibody positive patient with fibromyalgia. Thus, for 3 patients (to whom a level of anti-VGKC complex antibody of more than 100 pM was detected) among 4 anti-VGKC complex antibody positive patients with fibromyalgia in Example 1, a pain-relieving experiment by an anticonvulsant agent for an anti-VGKC complex antibody positive patient with fibromyalgia was conducted. These patients were patients with fibromyalgia of non-depression type.

To patient A, gabapentine 900 mg and clonazepam 0.5 mg were orally administered. As a result, the value of patient pain VAS was reduced to 40 based on 100 before administration.

To patient B, clonazepam 1.5 mg was orally administered. As a result, the value of patient pain VAS was reduced to 30 based on 100 before administration.

To patient C, gabapentine 1600 mg and clonazepam 1.0 mg were orally administered. As a result, the value of patient pain VAS was reduced to 20 based on 100 before administration.

From these results, it was found that the pain could be significantly relieved by an anticonvulsant agent in an anti-VGKC complex antibody positive patient with fibromyalgia. As described above, in the present invention, an appropriate treatment can be performed for fibromyalgia of an unknown cause when the anti-VGKC complex antibody was found to be a cause.

### Industrial Applicability

The present invention can be used in the clinical diagnosis service industry and in the pharmaceutical industry.

## Claims

1. A diagnostic agent for fibromyalgia relating to an antibody against a voltage-gated potassium channel, comprising:
a reagent for detecting an antibody against a complex of a voltage-gated potassium channel.

2. A diagnostic method for fibromyalgia relating to an antibody against a voltage-gated potassium channel, comprising:
determining whether a predetermined level or more of antibody is contained in a biological sample of a patient or not using a reagent for detecting the antibody against a complex of a voltage-gated potassium channel.

3. A therapeutic agent for fibromyalgia relating to an antibody against a complex of a voltage-gated potassium channel, comprising:
an anticonvulsant drug as an active ingredient in an effective amount.

4. The therapeutic agent in accordance with Claim 3,
wherein the anticonvulsant drug contains both or one of gabapentine and clonazepam.

5. The therapeutic agent in accordance with Claim 3,
wherein the anticonvulsant drug contains clonazepam.

6. The therapeutic agent in accordance with Claim 4 or 5,
being used for improvement of pain for a patient with fibromyalgia having an anti-VGKC complex antibody level of 100 pM or more in a measurement of anti-VGKC complex antibody level using serum.
